(19)

**Europäisches Patentamt**
**European Patent Office**
**Office européen des brevets**

(11)  **EP 4 762 946 A1**

(12)  **EUROPEAN PATENT APPLICATION**

(43) Date of publication:
    **24.06.2026   Bulletin 2026/26**

(21) Application number: **24222840.1**

(22) Date of filing: **23.12.2024**

(51) International Patent Classification (IPC):
    *A23L 29/256* (2016.01)    *A23L 29/262* (2016.01)
    *A23L 29/30* (2016.01)    *A23L 33/125* (2016.01)
    *A23L 33/16* (2016.01)    *A23L 33/00* (2016.01)
    *A23L 33/15* (2016.01)    *A23L 21/15* (2016.01)

(52) Cooperative Patent Classification (CPC):
    **A23L 29/256; A23L 21/15; A23L 29/262;**
    **A23L 29/30; A23L 33/125; A23L 33/15;**
    **A23L 33/16; A23L 33/40;** A61P 1/00

(84) Designated Contracting States:
    **AL AT BE BG CH CY CZ DE DK EE ES FI FR GB**
    **GR HR HU IE IS IT LI LT LU LV MC ME MK MT NL**
    **NO PL PT RO RS SE SI SK SM TR**

(71) Applicants:
    • **Kaunas University of Technology**
      **44249 Kaunas (LT)**
    • **UAB "Hydrocandy"**
      **21118 Trakai (LT)**

(72) Inventors:
    • **Leskauskaite, Daiva**
      **LT-44249 Kaunas (LT)**
    • **Kersiene, Milda**
      **LT-44249 Kaunas (LT)**

    • **Eisinaite, Viktorija**
      **LT-44249 Kaunas (LT)**
    • **Jasutiene, Ina**
      **LT-44249 Kaunas (LT)**
    • **Lazickaite, Enrika**
      **LT-44249 Kaunas (LT)**
    • **Judine, Kristina**
      **LT-21118 Trakai (LT)**

(74) Representative: **AAA Law**
    **A. Gostauto 40B**
    **03163 Vilnius (LT)**

Remarks:
    Amended claims in accordance with Rule 137(2)
    EPC.

(54)  **HIGH-WATER CONTENT FINGER FOOD**

(57)    The invention discloses a bite-sized high-water content finger food (FF) with a moderately thick interior contents consistency and an elastic membrane covering said interior contents so that it is resistant to an easy breaking when picked up with the fingers, yet, it melts in the mouth and is easily swallowed. The compositiion of the disclosed food, essentially, comprises a combination of ingredients: high water content, at least 90% by mass, e..g, in the range of 95-97%, and thick consistency providing agents: agar and sodium carboxymethylcellulose in an amount providing a shape-retaining gel texture, by at least 0,8% by mass of the agar with at least 0.5% by mass of sodium carboxymethylcellulose. Further, balanced proportions of nutrients (vitamins, microelements, flavours, color agents) can be included into the product composition. The product is useful as a nutritious food with high water content, providing efficiently nutrients and hydration to the elderly, helping to reduce/eliminate the risk of dehydration and aspiration pneumonia in the elderly.

Fig. 1

EP 4 762 946 A1

**Description**

FIELD OF INVENTION

The invention belongs to the field of food technology. More specifically, it is a one-bite-sized high-water content finger food (abbreviated: FF) with a moderately thick interior contents consistency and an elastic membrane covering said interior contents so that a single piece of the finger food is resistant to an easy breaking when picked up with the fingers, yet, it melts in the mouth and is easily swallowed.

BACKGROUND ART

About half of older people who need constant care are diagnosed with dehydration and about 25 percent of elderly people living in the community experience this disorder [1]. The most common consequences of dehydration are constipation, kidney failure, myocardial infarction, bedsores, poor wound healing, infections, adverse drug reactions, worse treatment outcomes, and worse quality of life. The most critical causes of dehydration are the following:

   1) a decrease in the sensation of thirst and the ability to retain sodium and water due to the deterioration of kidney function;
   2) decreased amount of water in the body due to decreasing muscle mass;
   3) chronic diseases: diabetes, hypertension, obesity, oral cavity problems (ulcers, dry mouth, prostheses), movement disorders, dementia, depression, speech disorders, vision disorders, dysphagia;
   4) acute conditions: fever, diarrhea, vomiting, trauma, emergency surgery, infections;
   5) certain medicines, e.g. laxatives;
   6) feeding problems: insufficient fluid intake, food with a lot of protein, salt, sugar and caffeine, little fruit and vegetables;
   7) social factors: social isolation, neglect, lack of support, dependence on caregivers, etc. [2].

Up to 50% of the nursing home residents suffer from another medical disability - dysphagia [3]. Dysphagia is a pathology that compromises the safety of swallowing and feeding in those who suffer from it [4]. Besides the impairment in dietary intake that will result in malnutrition, dysphagia also imposes stringent restrictions on the intake of water [5]. The use of thickened liquids is a suitable therapeutic strategy to reduce the risk of respiratory tract invasion in patients with dysphagia. There is also scientific evidence about the positive effect of thickened liquid therapy on the hydration status of patients with dysphagia [6]. However, some studies demonstrate that the consumption of only thickened liquids does not have a significant effect on the increase in water content in the body of these patients because patients who strongly dislike thickened liquids reduce the amount of fluid intake, so it is necessary to strictly monitor the amount of fluid intake in these patients [7]. Elderly people in nursing homes who require a modified texture diet have been found to consume significantly less fluid compared to those who drink regular fluids [8].

Psychological problems such as depression, isolation, cognitive impairment, dementia, and loss of ability to eat and drink independently are common in the elderly population, contributing to significantly reduced fluid intake [9]. It has been found that people with dementia often refuse or forget to drink. There are studies showing that Alzheimer's patients prefer foods that they can eat with their fingers alone rather than cutlery when it becomes too difficult to use them. The researchers behind these studies say that with this solution, Alzheimer's patients can gain independence and rediscover the joy of eating on their own. However, it is necessary to present attractive finger foods to ensure that these patients are willing to choose and consume them [10].

"Finger foods" refers to small, easily manageable food items that can easily be eaten without utensils and picked up with one hand [11]. These foods are designed to be easily handled and transported to the mouth. Finger foods are recommended by The European Society for Clinical Nutrition and Metabolism (ESPEN) [12]. Nutrition and geriatrics experts say that food specially prepared to be eaten with the fingers, easily grasped and transported from the plate to the mouth, can be beneficial for elderly people with cognitive disorders. Studies are being published proving finger food positively affects food consumption, primarily among people with dementia [13].

In recent years, finger foods have received several approvals as a suitable tool for texture-modified foods for dysphagia patients [14, 15, 16]. These handheld foods are typically of regular texture (crunchy or soft) and can also be adapted for mechanical soft textures-those are foods that are soft and can be easily chewed.
The International Dysphagia Diet Standardisation Initiative (IDDSI) developed standardized terminology and definitions for texture-modified foods and thickened fluids used in dysphagia management, which relies on textural and rheological properties to categorize different types of foods into eight levels (0-thin, 1-slightly thick, 2-mildly thick, 3-moderately thick/liquidized, 4-extremely thick/pureed, 5-minced & moist, 6-soft & bite-sized, 7-regular (easy to chew)) [17]. For people with swallowing disorders who are at risk of dehydration, fluid foods with better cohesiveness and lubrication at level 3 are usually required [18]. With reference to IDDSI categorisation, none of the currently designed finger foods meets this requirement, and most of them are levels 4 to 7.

There are also patent documents disclosing food products for similar purposes (e.g., confectionary) and with similar physical properties. According to the object of the invention, the closest patents to the present invention are the international application WO2020128531A1, with a priority date of December 23, 2019, and another international application WO2022208070A1, with a priority date of March 29, 2021. These patent documents describe gummies and gellies suitable for confectionery and therapeutic use, particularly in hydration therapies.

The international application WO2020128531A1 discloses a composition for preparing edible products comprising 0.2 wt% to 3 wt% of at least one polysaccharide gelling agent, the balance between water.

The international application WO2022208070A1 discloses a gelling composition for the preparation of edible products, which consists of Component A and Component B in a weight ratio of from 1 : 4 to 1.4 : 4. Component A comprises of at least one rigid gel-forming agent and Component B comprises at least one flexibility modifier. These inventions provide edible products containing a large proportion of water with a solid or semi-solid matrix. The edible products described in these patent documents are more accessible for the person to pick up and engage with a comparison with liquid or viscous sources of hydration. This is especially true for people with limited dexterity and coordination. However, the products' texture is unsuitable for people with swallowing problems.

The present invention aims to solve a different and specific problem and employs for that aim a product with a specific composition and physical properties.

SUMMARY OF INVENTION

**Problem to solve.** The present invention emphasizes a lack of food products and/or product groups that would:

- be handheld high-water content foods suitable for fluid replacement for the elderly people and patients at risk of dehydration;
- be easy to swallow and have a viscosity that be optimal for feeding the elderly and patients with mild or more severe swallowing disorders (dysphagia);
- have an attractive appearance - bite-size, easy to pick up by fingers and transport to the mouth; colors - bright, noticeable, taste and smell - expressive, fresh;
- have a sufficiently long shelf-life, retaining the micronutrients (vitamins, minerals).

**Solution.** The present invention discloses a specific product made of a special composition - one-bite high-water-content finger food (FF) with a moderately thick interior and an elastic membrane covering the interior so that it resists breaking when picked up with the fingers yet melts in the mouth and is easily swallowed. The composition of the product comprises a mixture of agar and sodium carboxymethylcellulose in an amount suitable to provide a shape-retaining gel texture with the description of level 6 soft and bite-sized foods according to IDDSI and has a viscosity of greater than 1000 cP.

The finger food is enriched with a complex of micronutrients, the nutritional value of which is more adapted or optimized for the elderly, taking into account changes in their appetite, taste perception, swallowing, and nutrient needs changes.

Nevertheless, this product can also be used by other people whose age and health conditions are not critical, so this product is already recommended for use.

Essential features of the invented product is a specific combination of ingredients in the product's composition:

- high water content, should be at least 90% by mass, preferably, above 95%, and with the technical feasibility of such product, in the range between 95% and 97%;
- consistency of the jelly composition, which is adapted for finger food consistency, having this high water content adapted for finger food consistency. In the view of so high-water contents, the consistency is defined by a sufficient amount of a jellying agent, specifically, the composition of agar and sodium carboxymethylcellulose in an amount suitable to provide a shape-retaining gel texture, by at least 0,8% by mass of the agar with at least 0.5% by mass of sodium carboxymethylcellulose;
- specific balanced proportions of further selected nutrients (vitamins, microelements, flavours, color agents), supporting the required shape-retaining gel texture and a sufficient shelf-life of the product.

Production method. Furthermore, the invention also involves and discloses a method of preparing or producing the aforementioned finger food product. The method comprises such general stages:

- preparation of dry raw materials (essential ingredients) mixture,

- making a formulation (suspension) for the FF, by blending all essential raw materials (except vitamins),
- pH adjustment,
- heat treatment, for dissolving the components and ensuring microbial safety,
- optionally, addition of vitamins;
- shaping a bite-sized FF product and gel formation,
- optionally, packaging and storage of the obtained FF product.

**Effects of invention.** The effect or outcome of the present invention is the easy-to-swallow and shape-retaining texture of the one-bite food that resists breaking when picked up with the fingers. The overall stability of the micronutrient enriched high water-content finger food is determined by keeping it for 30 days. The release kinetics of micronutrients were investigated during the *in vitro* digestive degradation of the food, confirming good digestion of the product and gradual release of the nutrients during the simulated G0-G60-G120 digestion period. Simulated gastrointestinal activity is used in many food and nutrition sciences areas because human testing is often expensive, resource-intensive, and ethically controversial. In developing foods for elderly consumers, the *in vitro* digestive degradation and characterization of micronutrients released from food are essential to assess the product's properties up to the final phase of its assimilation by the body.

<u>Uses and applications.</u> The present invention finds the following specific efficient uses and applications:

- The use of thickened liquids is a common therapeutic strategy to reduce the risk of respiratory tract invasion in patients with dysphagia. However, the consumption of only thickened liquids does not significantly affect the increase in water content in the body of these patients because patients who strongly dislike thickened liquids reduce the amount of fluid intake. The solution is a handheld food product containing high water content, thereby suitable as a fluid replacement for people at risk of dehydration.
- The elderly prefer foods they can eat with their fingers alone rather than cutlery when using them is too difficult. Using utensils (cups, drinking glasses, straws, etc.) cannot be avoided when consuming liquids. The solution is one-bite finger food that can easily be eaten without utensils and picked up with one hand.
- The elderly can gain independence and rediscover the joy of eating independently by consuming finger food. However, it is necessary to present attractive finger foods to ensure that these patients are willing to choose and consume them. The solution is a pleasing appearance - bite-size, easy to pick up by fingers and transport to the mouth; colors - bright, noticeable, taste and smell - expressive, fresh.
- Micronutrients (vitamins and minerals) are necessary for the body's metabolism and vital functions, and as they age, their deficiency adversely affects the physical and mental functions of the body. The amount of micronutrients in this finger food ensures that the daily requirement of nutrients is satisfied when eating these foods, so there is no need to use vitamin and mineral preparations and food supplements.

DESCRIPTION OF DRAWINGS

Some essential aspects of the invention are explained in the drawings and graphs. The drawings and graphs are an integral part of the detailed description and are provided as a reference to a possible implementation or visual explanation of the invention, but not limiting the scope of the invention.

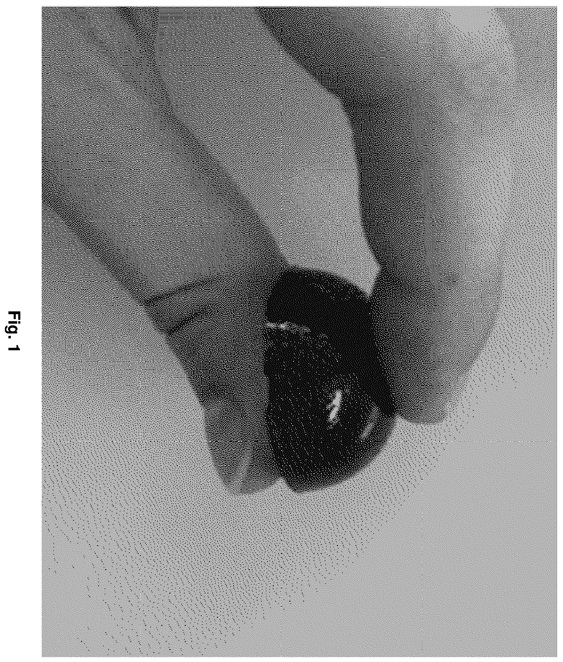

**Fig. 1.**    A general view of the invented finger food product in use.

**Fig. 2.**    Testing results of finger foods (FFs) with different amounts of agar according to IDDSI (International Dysphagia Diet Standardization Initiative) framework: **a)** FF with 0.8% of agar; **b)** FF with 1.0% of agar; **c)** FF with 1.5% of agar.

**Fig. 3.**    Rheological performance of FFs with different amounts of agar. (A) Shear yield stress (B) Shear rate dependence of viscosity. (C) The thixotropic recovery evaluated by the shear viscosity as a function of time at 0.1 $s^{-1}$ for the first and the last 3 min and 50 $s^{-1}$ for the medium 1 min. (D) Strain-dependence of storage modulus (G') and loss modulus (G") with a frequency of 1 Hz. (E) Frequency-dependence of storage modulus (G') and loss modulus (G") with a strain of 1 %.

**Fig. 4.**    Appearance of products under the three-step "low-high-low" recovery step.

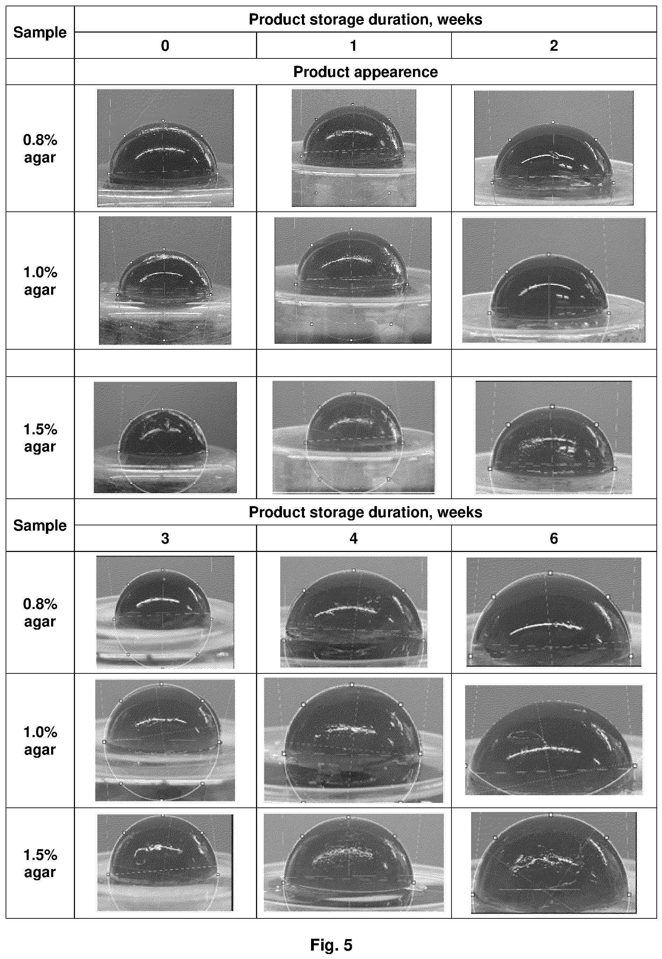

**Fig. 5.**    Appearance of the shelf life of products, taken after 0, 1, 2, 3, 4, and 6 weeks, from the production moment.

DETAILED DESCRIPTION OF INVENTION

The present invention provides a manufacturing approach that gives an easy-to-swallow and shape-retaining texture of the one-bite finger food (FF) designed to be easily handled and transported to the mouth.

**Nutritional values of the FF product.** The nutritional composition and texture of the high-water content finger food (FF) comprise the following elements/ingredients and proportion ranges (in mass) thereof:

- 95.0 - 97.0 g/100 g of water;
- 3 - 4.5 g/100 g of total carbohydrates, of which sugars 2.0 - 3,0 g/100 g;
- Fibers 0.5÷0.6 g/100 g;
- Proteins <0.5 g/100 g;
- Salt 0.09÷0.12 g/100 g;
- 0.07 - 0.08 g/100 g of vitamin C; 88÷100 RI*%;
- 0.20 - 0.25 mg/100g of vitamin B9; 80÷100 RI%;
- 2.4 - 2.5 $\mu$g/100g of vitamin B12; 96÷100 RI%;
- 4.0 - 5.0 $\mu$g/100g of vitamin D3; 80÷100 RI%;
- 50-55 $\mu$g/100g of selenium; 91÷100 RI%;
- 8-10 mg/100g of zinc; 80÷100 RI%;
- 12-14 mg/100g of iron; 86÷100 RI%.

*- RI Reference Intake (RI) communicating recommended nutrient intake to the public.*

The consistency of the high-water content FF corresponds to level 6 (soft and bite-sized) according to the International Dysphagia Diet Standardization Initiative (IDDSI) Framework. The viscosity is higher than 1000 cP.

**Components of the high-water content finger food.** The components listed below and their amounts compose one of the possible product embodiments developed and tested in the laboratory. However, the following components and their amounts do not limit the present invention, both in terms of the selection of the ingredient materials and their proportions of the composition. For example, in different embodiments - either water with saccharides and organic acids can be blended, or otherwise, a water can be mixed with a natural juice concentrate (comprising a part of the required water, saccharides and natural organic acids).

<u>Essential chemical components/ ingredients</u> included in the composition of the produced FF products are:

- water at least 95%, or in the range from 95% to 97% by mass;
- mono- and disaccharides in range from 1.2 to 2.0 % by mass; there can be a mixture of mono- and disaccharides within this percent range;
- organic acids - in range from 0.5 to 0.9 % by mass; organic acids selected from citric, malic, quinic acids, or there can be a mixture of any of those organic acids within this percent range;
- agar - at least, 0,8%, or in range from 0,8% to 1,5% by mass;
- sodium carboxymethylcellulose - at least, 0,5%, or in range from 0,5% to 0.6% by mass;
- acidity regulation compound, as much as needed to maintain pH 3.1-3.2 of the prepared FF composition.

The <u>essential components/ ingredients</u> in the composition, alternatively, can be composed as the following ingredients and mass/weigth percentages thereof:

- water, at least 71 %, or in range from 71% to 78% by mass;
- juice concentrate (dry materials in the juice concentrate is 9=10% by mass), where the juice concentrate is included in range from 18 to 27 % by mass in the mixture of the FF composition;
- agar - at least, 0.8%, or in range from 0.8% to 1.5% by mass;
- sodium carboxymethylcellulose - at least, 0.5%, or in range from 0.5% to 0.6% by mass;
- acidity regulation compound, as much as needed to maintain pH 3.1 - 3.2 of the prepared FF composition.

<u>Complementary nutritious components</u> can be added into the composition of the product, thereby improving the nutritional function of the FF products according to the present invention. Those complementary components are any or a composition of:

- vitamin C,
- vitamin B9,
- vitamin B12,

- vitamin D3,
- zinc, iron, and selenium.

The complementary nutritious components may be added to the composition of FF in quantities such that they correspond to 100% of the reference intake (RI - Reference Intake - recommended intake of nutrients for the population) per 100 g of product, and the total amount may not exceed 0.8% by weight.

Colorants and flavors can be added to the FF product composition, preferably of natural origin, as much as needed. The amounts of the colorants and flavors are usually small, and they do not affect the proportions of other substantial components/ingredients, the FF composition, and the essential properties of the obtainable FF product.

Preservative components can be included in the composition of the FF product; those are Potassium sorbate and Sodium benzoate. The preservative ingredients may be included with the aim of giving the FF product a longer shelf life and a longer storage duration of up to 4 weeks. In an embodiment of the invented product, storage for up to 4 weeks may be intended without substantial changes in the product's appearance and physical and nutritional properties; this is ensured by the aforementioned preservative components.

Otherwise, without including the preservative components, the obtained FF product can retain its physical and nutritional properties until 1 week. The amounts of the preservatives into the FF composition selected in the ranges:

- Potassium sorbate - in range from 0.05 to 0.1 % by mass;
- Sodium benzoate - in range from 0.01 to 0.05 % by mass.

**The general workflow of high-water content FF product preparation** comprises stages of:

- preparation of the essential dry raw materials (saccharides, organic acids, agar, sodium carboxymethylcellulose) mixture and the required water contents,
- making a liquid formulation (suspension), by blending the essential FF raw materials (except vitamins);
- pH adjustment of the blended liquid formulation (suspension), by adding the acidity regulator;
- heat treatment of the liquid formulation (suspension), for dissolving the components and ensuring microbial safety;
- optionally, cooling the formulation to a temperature suitable for further adding complementary components, addition and blending in the complementary components: vitamins and other nutritional components;
- shaping the above processed formulation into bite-sized product shapes, and allowing gel formation from the shaped liquid formulation, thereby, obtaining the final ready-to-eat FF product;

and further, optionally:

- packaging and storage of the obtained product.

**Technical workflow (instructions) of the FF product preparation.** More specifically, example embodiments of the high-water content finger food preparation are described as follows:

- Preparing the mixture of dry raw materials:

  ◦ **for FORMULATION I:** Dry raw materials are weighed and thoroughly mixed in the following amounts: agar (selected from 0.8 - 1.5 % by mass), sodium carboxymethylcellulose (selected from 0.5% - 0.6 % by mass), mixture of mono- and disaccharides (selected from 1.2 - 2.0 % by mass), mixture of organic acids (citric, malic, quinic) (selected from 0.5 to 0.9 % by mass), optionally, colorants and flavors, preferably of natural origin (as much as needed), minerals (zinc, iron, selenium) (in the amount to meet 100 % of RI) product), sodium benzoate (0.01 - 0.05 % by mass) and potassium sorbate (0.05 - 0.1 % by mass).
  ◦ **for FORMULATION II:** Dry raw materials are weighed and thoroughly mixed in the following amounts: agar (0.8 - 1.5 % by mass), sodium carboxymethylcellulose (0.5% - 0.6 % by mass), minerals zinc, iron, selenium) (in the amount to meet 100 % of RI product), sodium benzoate (0.01 - 0.05 g% by mass) and potassium sorbate (0.05 - 0.1 % by mass).

- Making a liquid formulation for finger food, by blending all raw materials:

  ◦ **for FORMULATION I:** The formulation is made by mixing and homogenizing the dry raw materials with water (95 - 97 % by mass).
  ◦ **for FORMULATION II:** The formulation is made by mixing and homogenizing the dry raw materials with water (71 - 78 % by mass). After that, juice concentrate (18-27 % by mass) is added and thoroughly mixed.

- Adjusting pH. The pH of the formulation has to be adjusted to pH 3.1 - 3.2. If pH adjusting to 3.1 - 3.2 is needed, the pH is adjusted by adding citric acid until the pH of the formulation reaches pH 3.1 - 3.2.
- Heating. The formulation is heat treated, for dissolving the components and ensuring microbial safety, at 98 - 100 °C for no longer than 5 minutes. After that, the formulation is cooled down to 54 - 56 °C.
- Adding vitamins. Vitamins (C, B9, B12 and D3) are weighed and thoroughly mixed with the formulation in the amount to meet 100 % of RI product),
- Shaping the prepared formulation (I or II) into a single-bite-sized product shape (shaped mold) and allow gel formation. The formulation is poured into molds and kept at 2-6 °C until a gel forms, but not less than 12 hours.

**Physical characteristics of the product.** The following **characteristics** of the high-water content finger foods were examined:

pH was measured using a WTW digital pH meter 3110 (*WTW,* Weilheim, Germany) with penetrating probe N 1048A.

The contact angle was determined from the photographs taken with a high-resolution digital camera. Photographs were analyzed using imaged software with the Contact Angle Plug-in to obtain θ, which was used to calculate the contact angle.

Rheological characteristics were evaluated by the swallow-related shear sweep, amplitude sweep, and frequency sweep tests at 25°C using a rheometer with a plate-to-plate system (diameter 20 mm, gap 1 mm) 24 h post-processing.

*The shear-dependent viscosity was* estimated over a shear ranging from 0.01 to 100 $s^{-1}$ to evaluate the shear viscosity at 50 $s^{-1}$ ($\eta_{50}$).

*The strain-dependent viscoelasticity* was measured in the linear viscoelastic (LVE) region. The oscillation amplitude sweep test was accomplished over a strain range from 0.01 to 1000 % and a fixed frequency of 1 Hz to evaluate the strain-related storage modulus (G'), loss modulus (G''), and loss tangent (tan $\delta$).

*The frequency-dependent viscoelasticity* was determined in the linear viscoelastic (LVE) region. The storage (G') and loss modulus (G'') were measured, over the angular frequency range from 0.1 to 100 rad/s.

*Shear recovery behavior* was evaluated by a three-step "low-high-low" flow sweep test. The test was run in tree flow peak hold: 0.1$s^{-1}$ for 3 min, 50 $s^{-1}$ for 1 min, and 0.1$s^{-1}$ for 3 min.

*The shear yield stress* was determined by performing a flow sweep over a shear ranging from 0.001 to 0.1 $s^{-1}$. The initial peak in shear stress was called the shear yield stress.

Texture profile analysis (TPA) was performed on a texture analyzer (TA.XT Plus, Stable Microsystem Ltd, Godaiming, UK). The P/10 10 mm diameter cylinder Perspex probe had a pre-test speed of 1.7 mm/s, a test and return speed of 2.5 mm/s, and a target distance and duration between 4 mm and 5 s cycles, respectively. Two subsampling units were tested and averaged per replicate to record the hardness, cohesiveness, and adhesiveness values.

NDD testing was carried out to categorize the samples according to the NDD levels. The shear-dependent viscosity was estimated over a shear ranging from 0.01 to 100 $s^{-1}$ to evaluate the shear viscosity at 50 $s^{-1}$ ($\eta_{50}$).

IDDSI testing methods were used to categorize the samples according to the IDDSI levels (The International Dysphagia Diet Standardisation Initiative 2019). The fork pressure test and spoon pressure test were employed.

**Sensory evaluation of the product by tasting participant groups.** Sensory evaluation was conducted in the nursing and supportive care hospital Addere Care, Lithuania. Participants were eligible if they were 60 years old or older. Participants were excluded if they were allergic or intolerant to one of the foods of the study if they had severe dementia (i.e., MMSE test < 10), with marked respiratory failure, clear gastrointestinal pathology (exacerbation of ulcers, diarrhea, nausea, severe dysphagia with a high risk of aspiration). The sample size was calculated to detect a difference of 0.8 on the 7-point scale. Considering an average Standard Deviation (SD) of 1.6 liking score in older people (Sulmont-Rosse et al., 2018), a minimum of 51 participants was required (power=0.80; $\alpha$ = 0.05).

Participants took part in one individual session from 10:00 to 13:00. Participants were served three products in a monadic sequence. The presentation order of the products was balanced across participants. Products were served at room temperature on a plate. Participants were informed that they would taste cherry-flavored, citric-flavored, and quinces-flavored products. However, they were unaware of the product's manufacturing process or nutritional information. Participants were asked to taste each product and complete a questionnaire including nine questions on liking, comfort, and texture. Participants were asked to answer each question on a 7-point categorical scale labeled at each anchor. Questions were the following:

- This product is?... - "Difficult to handle by hand" / "Easy to handle by hand";
- How much do you like the colour / the feeling in the mouth? - "I totally dislike" / "I totally like";
- The taste and aroma of this products is?... - "Weak" / "Strong";
- This product is?... - "Soft" / "Firm";
- This product is?... - "Dry / Juicy";
- This product is?... - "Difficult to swallow" / "Easy to swallow";

- The aftertaste of the product during swallowing is?... - "Weak" / "Strong" and "Not mouth coating" / "Very mouth coating";

After each product, participants were asked to rinse their mouths with plain water. The tasting session was carried out in a hospital ward or meeting room.

<u>Imitating digestion _(in-vitro)_ of the high-water content finger food.</u> Simulated _in vitro_ digestion was performed with the prepared high-water content finger foods to evaluate the release of micronutrients in the targeted places of the gastrointestinal tract. A non-gelled solution with the same amount of micronutrients was used as a control. The study was performed according to the static _in vitro_ digestion protocol Infogest. Three test tubes were used to obtain the digestion profile, and in them, the digestion process was stopped after 0, 60, 120, and 180 minutes in the gastric phase (G0, G60, G120, and G180), and 60 and 120 minutes in the intestinal phase (D60, D120), respectively. Gastric phase samples were neutralized to pH $7.0 \pm 0.1$, and the digestive process of intestinal phase samples was stopped by cooling in ice water to 0-4°C. After the reaction was stopped, the samples were centrifuged at 4000rpm. in +4°C temperature and filtered. The soluble digestive fraction was collected, frozen, and stored at -18°C until analysis. The content of vitamins and minerals released into gastrointestinal fluids was analyzed. The digestion procedure was performed twice.

<u>Stability of product characteristics during shelf-life.</u> The following characteristics of the high-water content finger food during storage at +4°C for 42 days were examined: pH, contact angle, weight loss, hardness, G', and vitamin stability.

The weight loss was determined as the weight change of each finger food during storage and calculated as the percentage of pre-storage weight:

$$Weight\ loss = ((m_1 - m_2) / m_1)\ x\ 100,$$

where $m_1$ is the sample weight (g) before storage, and $m_2$ is the finger food weight (g) after a certain storage period. Each measurement was performed with 5 finger foods.

Vitamins stability was calculated using the following equation:

$$Stability = (C_{in} - C_f) / C_{in}\ x\ 100\%$$

where $C_{in}$ is the amount of a particular vitamin initially added to the product, $C_f$ - is the amount of a particular vitamin found in the product after 42 days of storage.

The pH factor and textural properties were determined as described earlier.

**Results.** The developed FFs are described in several essential aspects: textural and rheological properties, physicochemical changes during storage, and micronutrient release degradation during _in vitro_ digestion simulation. NDD and IDDSI tests and sensory analysis were used to confirm that the high-water content finger foods met the requirements for dysphagia-friendly food.

**Table 1. Textural characteristics of high-water content finger foods.**

| Agar content in finger food, % | pH | Textural properties | | | Rheological properties | |
|---|---|---|---|---|---|---|
| | | Hardness, g | Adhesion, g.s | Cohesive ness | G', Pa | tanδ |
| 0.8 | $3.36 \pm 0.01A$ | $144.3 \pm 10.13A$ | $-13.01 \pm 0.54A$ | $0.12 \pm 0,01A$ | $1839 \pm 0.00A$ | 0.08A |
| 1.0. | $3.17 \pm 0.01B$ | $187.47 \pm 31.48B$ | $-11.79 \pm 0.92A$ | $0.13 \pm 0.01A$ | $1849 \pm 0.00A$ | 0.08A |
| 1.5 | $3.20 \pm 0.01B$ | $368.15 \pm 59.05C$ | $-10.61 \pm 0.44B$ | $0.12 \pm 0.01A$ | $6473 \pm 298.96B$ | 0.09B |
| _Values are presented as mean±standard deviation; Uppercase letters indicate significant (p<0.05) differences in the characteristics of FF with different agar content._ | | | | | | |

**Table 2. High-water content finger foods classification as products indicated for people with dysphagia**

| Agar content in finger food, % | NDD* testing results | IDDSI** testing results |
|---|---|---|
| 0.8 | Level III ($\eta_{50}$=1096.21 mPa*s), honey-like | Level 6, soft, one bite size, views in Fig. 1 a |
| 1.0 | Level IV ($\eta_{50}$=1949.85mPa*s), pudding-like | Level 6, soft, one bite size, views in Fig. 1 b |

(continued)

| Agar content in finger food, % | NDD* testing results | IDDSI** testing results |
|---|---|---|
| 1.5 | Level IV ($\eta_{50}$=5538.25mPa*s), pudding-like | Level 6, soft, one bite size, views in Fig. 1 c |
| * - The National Dysphagia Diet Task Force<br>** - The International Dysphagia Diet Standardisation Initiative | | |

Table 1 discloses the textural characteristics of the FFs with different agar content (0.8-1.5 %). Instrumental texture analysis was performed to simulate the chewing behavior of the FFs. Hardness, adhesiveness, and cohesiveness were evaluated. Agar added had an influence on the hardness of FFs and had no significant effect on the adhesiveness and cohesiveness of finger foods. The hardness was in the range of 144.3 ± 10.13 - 368.15 ± 59.05 N. Such low hardness values suggest that finger foods will be easy to bite and squeeze between the tongue and palate. Cohesiveness values show the internal strength of food bolus. Low cohesiveness (0.12±0.01 - 0.13±0.01) helps prevent the product's sudden disintegration during swallowing, which is important for safe swallowing. All FFs showed similar low adhesiveness values from -10.61 ± 0.44 to 13.01 ± 0.54. Foods with low adhesiveness values require less time to form a suitable bolus. Therefore, the swallowing of such foods can not be accompanied by the risk of choking.

The categorization of FFs within the NDD and IDDSI levels is presented in Table 2. As the agar concentration increased, the $\eta_{50}$ values of FFs increased. This categories FFs with 0.8 % of agar as honey-like (350-1750 mPa s) and FFs with 1.0 % and 1.5 % of agar as spoon-thick or pudding-like (> 1750 mPa s) dysphagia-friendly products.

A fork pressure test, spoon tilt test, and fork separation test were performed as well. The results were similar for all FFs. When finger foods were pressed with the tines of a fork or spoon they squashed, broke apart, and did not return to their original shape when the fork or spoon was removed. Therefore, FFs can be characterized as level 6-soft & bite-sized foods according to IDDSI. That means they require chewing, but not biting, tender and moist without visible liquid separation.

Taking food by hand is very important for older people who have difficulty handling cutlery (apraxia) or remaining at the table for the entire duration of a meal. People with apraxia disorders use their fingers when eating, even if the food form is not suitable for that. Sensory analysis results of FF with 1.5% agar are presented in Table 3. The study involved 62 participants - 22 men and 40 women, whose ages ranged from 47 to 96 years (average age 78 years). Also, 7 study participants had dysphagia, which accounts for 11.3% of all respondents. According to the results obtained, FF was evaluated as easy to pick up by hand since as many as 51.6% of respondents rated FFs with the highest score (7/7). The color of these products was rated 6.03 out of a possible 7 points, and the mouthfeel was rated by assigning more than 5 points. When assessing the softness of these products, the results showed that 40% of respondents rated the FF as very juicy. The survey results showed that the products are easy to swallow, with an average score of 6.23 out of 7 possible. Participants with dysphagia gave an average of 6.5 points for FFs for ease of swallowing. The aroma intensity and aftertaste were assessed as acceptable, with the evaluators giving an average of 3-4 points. The property, which is associated with the instrumental method - adhesiveness, is the feeling in the mouth, which was also assessed as uncharacteristic for this product, i.e., about 6 points were assigned (7 does not feel in the mouth at all).

### Table 3. Results of sensory evaluation of high-water content finger foods

| Question | Evaluation |
|---|---|
| Difficulty to take by hand | 6.00±1.34 |
| Liking of the color | 6.03±1.38 |
| Liking of the mouth feeling | 5.50±1.83 |
| The intensity of the taste and aroma | 2.67±1.83 |
| Firmness | 5.08±1.79 |
| Juiciness | 4.28±1.81 |
| Swallowing difficulty | 6.23±1.42 |
| Afterfeel intensity | 3.93±1.89 |
| Mouth couting after-feel | 5.82±1.60 |

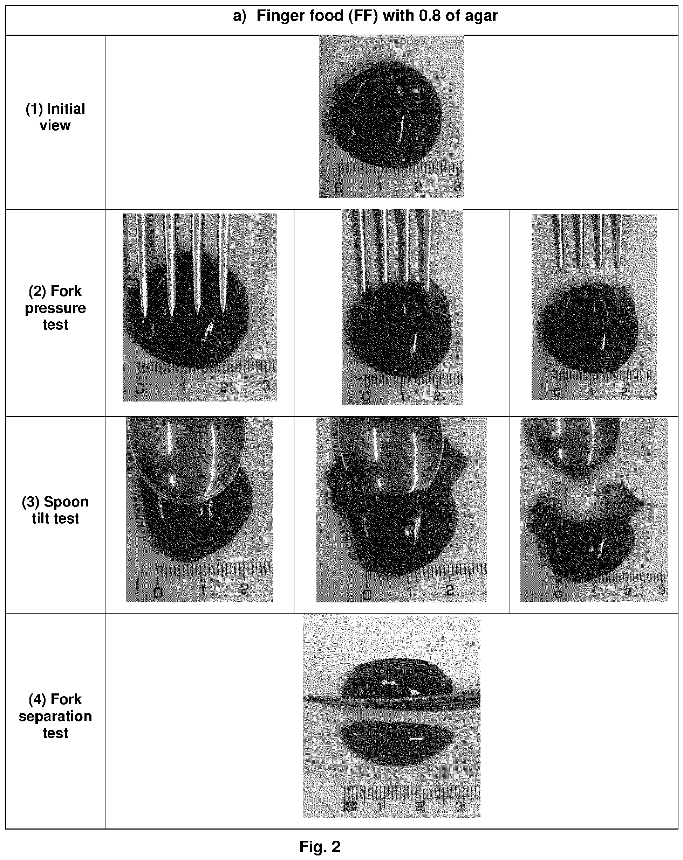

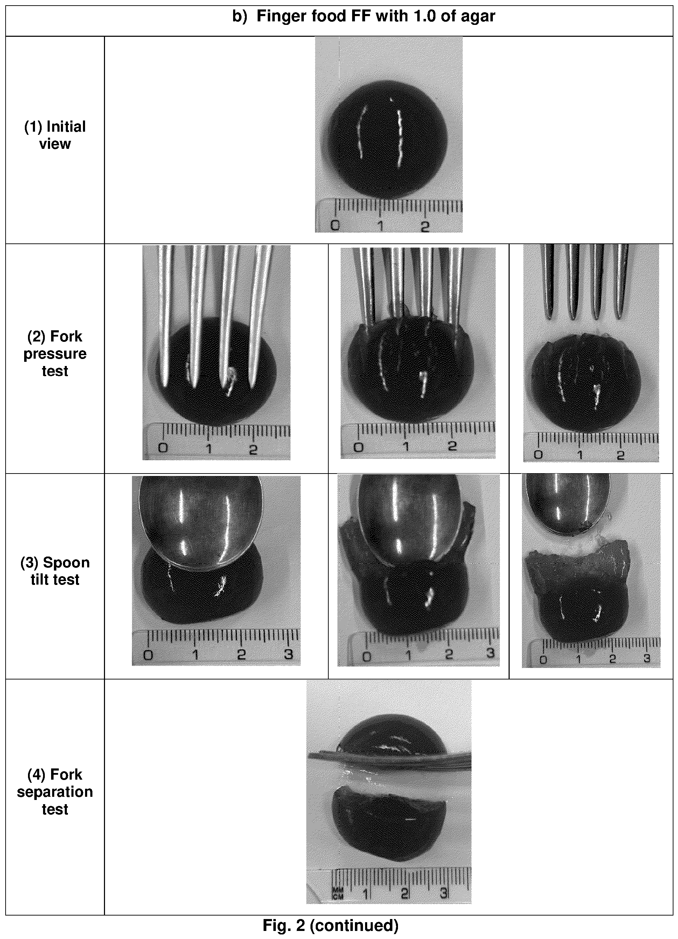

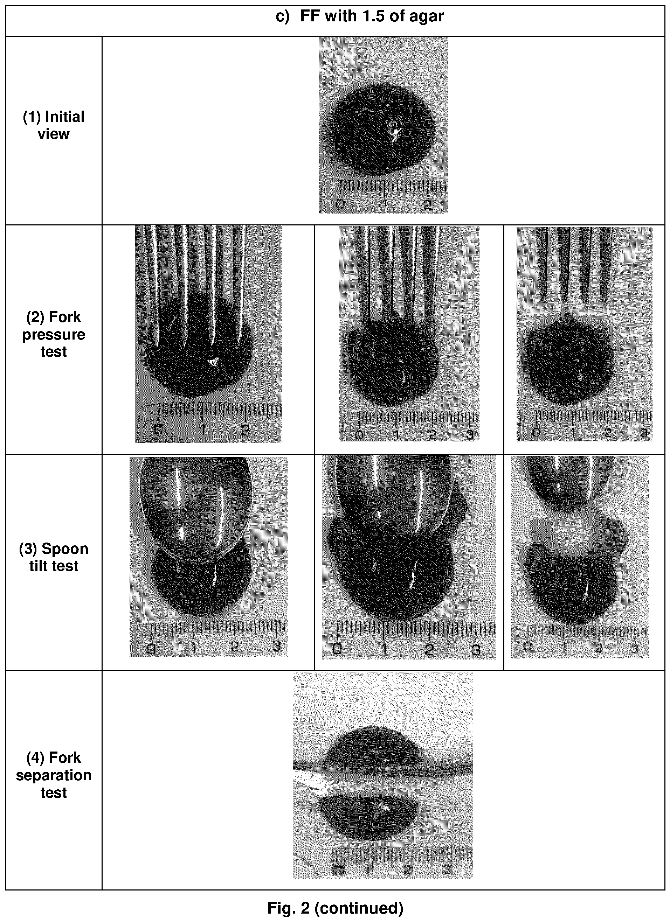

The rheological performance of high-water content finger food made with different agar content during swallowing is presented in Fig. 2. The rheological properties, including shear viscosity, primarily depend on the competitive balance between gel formation kinetics and shear strength, which is mainly controlled by the concentration of gelling agents. The shear sweep test showed a trend of increasing shear yield stress and $\eta_{50}$ with increasing agar concentration from 0.5% to 1.5%, providing rheological evidence for the shear thinning behavior when viscosity dramatically declined with the increase in shear rate (Fig. 2 B). FF with 0.8 and 1.0 % of agar showed smaller shear yield stress (46.23 Pa, 89.32 Pa) in comparison with that of FF with 1.5 % of agar (234.59 Pa), which is essential for easily sheared flow during swallowing (Fig. 2 A). Despite that, FFs with 1.0 and 1.5 % of agar exhibited thickness level 4 according to $\eta_{50}$ range of NDD levels for thickening liquids ($\eta_{50}>1750$ mPa.s, pudding-like) (Table 2). This means that FFs 1.0 and 1.5 % of agar are appropriate for people with severe dysphagia, considering that $\eta_{50}$ corresponds to the shear rate in oral cavities made during swallowing.

Subsequently, these findings were further confirmed by the frequency and strain-dependent dynamic viscoelastic test. All samples had significantly higher G' than G", and both appeared to be quite flat over a broad frequency spectrum ( Fig. 2E), indicating that at rest, all samples are in the solid-state gel form. This finding aligned with IDDSI testing results that all FFs were classified as soft, one bite size. With the increase in agar concentration, an increasing trend was observed for the initial G' (Table 1).

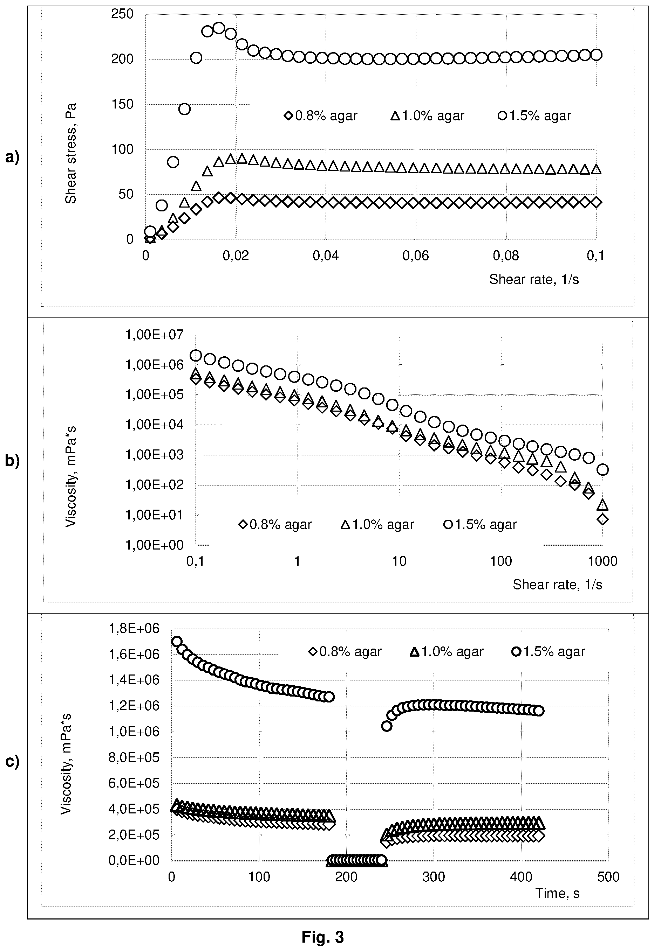

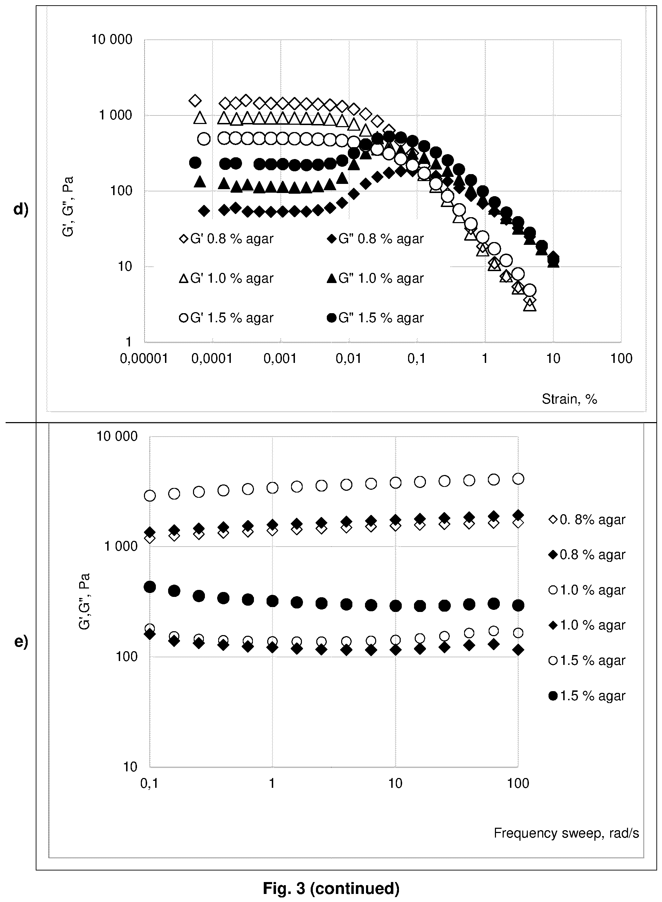

To further verify the behavior of the FFs during swallowing, a deformation-viscoelasticity profile (amplitude sweep) was determined, which allows the identification of the LVR within which G' and G" remain constant (Fig. 2D). When the strain exceeded the LVR, the gel structure began to break down, which is an indication of the gel transitioning from a solid to a liquid state. This property is crucial for facilitating ease of swallowing. As shown in Fig. 2D, no differences in the LVR length were observed, indicating that a similar strain was needed for all FFs to start a solid-fluid transformation. Three-step "low-high-low" was then performed to assess whether the FFs tended to recover quickly after shearing during a simulated oral shearing process (at a shear rate 50 s$^{-1}$) (Fig. 2C, 3). The shear recovery tests showed a declining recovery factor in the peak viscosity of samples (from 75% to 49%) as agar concentration was increased from 0.8% to 1.5%. It could be understood that increasing agar concentration caused an increase in weak interactions between gelling agent molecules. These weak interactions contributed to the solid-like behavior when subjected to lower yield stress and the fluid behavior when these interactions were broken at higher yield stress.

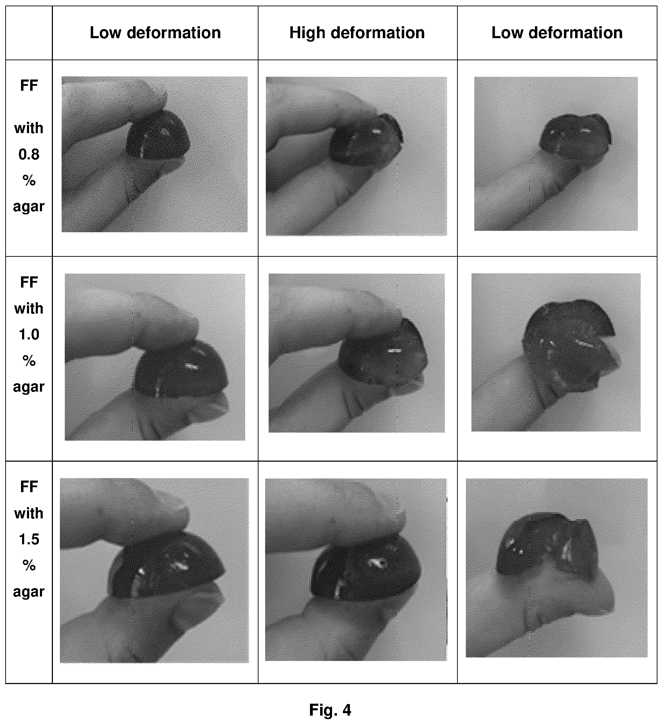

Physical stability, rheological properties, pH, and weight loss of FFs with different amounts of agar were examined during storage at +4 C (Table 4). During storage, a slight decrease in pH values and a significant increase in hardness and G' values during storage were observed in all tested FFs. Changes in the textural characteristics could be influenced by a marked increase in weight loss during storage. Weight loss is essential for product stability during storage. This index was dependent on the agar concentration and storage duration. As agar concentration was increased from 0.8% to 1.5%, the weight loss was significantly lower during storage. The maximum weight loss was observed for the FFs with 0.8% of agar after 6 weeks of storage - 14.11$\pm$3.30 %. Meanwhile, FFs with 1.5% agar lost 5.98$\pm$2.67% of their weight after 6 weeks of storage. Although the weight loss during storage, no water release was observed (Fig. 4).

The contact angle tests the quality of surfaces by characterizing the wettability of a material. We used this characteristic to evaluate how FFs maintain shape on contact with the surface. The significant changes in contact angle were determined for the FFs with a lower amount of agar (0.8%) after 6 weeks of storage. FFs containing 1.0% of agar were characterized by slight changes in contact angle after 4 weeks of storage and a significant decrease up to 82.0° after 6 weeks of storage. No changes of contact agle were detected during the storage of FFs with 1.5% of agar. So, we can conclude that FFs with 1.5% agar can maintain their shape during storage for 6 weeks, FFs with 1.0% agar - during storage for 4 weeks, and FFs with 0.8% agar - during storage for 1 week.

**Table 4. Properties of FFs during storage (up to 6 weeks).**

| Sam pie | Storage duration, weeks | | | | | |
|---|---|---|---|---|---|---|
| | 0 | 1 | 2 | 3 | 4 | 6 |
| **pH** | | | | | | |
| **0.8% agar** | 3.36±0.01a A | 3.21±0.01b A | 3.11±0.00c A | 3.08±0.02c dA | 3.05±0.01d A | 2.97±0.01e A |
| **1.0% agar** | 3.17±0.01a B | 3.09±0.01b B | 3.07±0.00b cB | 3.04±0.02c dA | 2.99±0.03d B | 2.91±0.03e B |
| **1.5% agar** | 3.20±0.01a B | 3.17±0.01b C | 3.12±0.00c A | 3.12±0.00c B | 3.08±0.01d C | 3.01±0.01e C |
| **Hardness, N** | | | | | | |
| **0.8% agar** | 115.3 ± 4.95aA | 115.81 ±9.48aA | 144.89± 0.13bA | 143.95±33. 64bA | 154.23±15. 58bA | 139.45±18. 48bA |
| **1.0% agar** | 94.98 ± 13.54aA | 87.47 ± 31.42aA | 104.66±17. 02aB | 92.15 ± 20.94aA | 84.09 ± 11.81aB | 81.01 ± 19.23aB |
| **1.5% agar** | 230.99±62. 88aB | 368.15±59. 09bB | 340.71±50. 42bC | 382.23±27. 07bB | 424.13±32. 12bC | 339.69±37. 76bC |
| **G', Pa** | | | | | | |
| **0.8% agar** | 1392.90 ± 0.00aA | 1839.20 ± 200.11bA | 1846.00 ± 98.57bA | 1864.55 ± 125.794bA | 1716.60 ± 160.09cA | 1856.00 ± 150.19bA |
| **1.0% agar** | 1567.80 ± 0.00aB | 1849.70 ± 308.86bA | 1746.30 ± 0.00cA | 1869.25 ± 173.59bA | 2008.05 ± 143.75dA | 1903.05 ± 20.57bA |
| **1.5% agar** | 3394.90 ± 298.96aC | 6473.15 ± 427.89bB | 6969.65 ± 795.71dcB | 6925.80 ± 751.80cB | 6859.45 ± 589.94cB | 7128.35 ± 350.51dB |
| **Weight loss, %** | | | | | | |
| **0.8% agar** | - | 4.72±1.07a A | 7.94±0.69b A | 9.77±1.53b cA | 12.44±2.71 cdA | 14.11±3.30 dA |
| **1.0% agar** | - | 2.92±0.77a AB | 5.73±0.33b B | 6.52±0.94b dB | 7.62±1.19d cB | 8.95±1.22c B |
| **1.5% agar** | - | 1.90±0.41a B | 2.87±0.82a bC | 4.24±2.11a bB | 5.05±2.08b B | 5.98±2.67b B |
| **Contact angle, °** | | | | | | |
| **0.8% agar** | 86.3 ± 0.31aA | 83.1 ± 2.00bA | 83.8 ± 1.38bA | 84.2 ± 1.51bA | 80.9 ± 0.71 cA | 83.1 ± 0.07bA |
| **1.0% agar** | 87.7 ± 1.73aA | 84.7 ± 2.40aA | 83.2 ± 2.82aA | 87.7 ± 1.61aB | 90.4 ± 2.40aB | 83.0 ± 0.28bA |
| **1.5% agar** | 86.6 ± 1.48aA | 85.5 ± 1.47aA | 85.7 ± 1.56aA | 85.7 ± 0.83aAB | 85.6 ± 0.75aC | 86.4 ± 0.21aB |
| Values are presented as mean±standard deviation; Lowercase letters indicate significant ($p<0.05$) differences in the characteristics of FF during storage; Uppercase letters indicate significant ($p<0.05$) differences in the characteristics of FF with different agar content | | | | | | |
| **Product appearence** | | | | | | |
| **Pictures of the product provided in Figure 4** | | | | | | |

To obtain a therapeutic benefit from the FFs enriched with micronutrients, these compounds must be stable during the technological process and have a shelf-life. This study used the retention of added vitamins after 6 weeks of storage at 4°C to indicate FF stability (Table 5). The highest retention was determined for vitamin B9, there was no loss of this vitamin during the study period. The retention of other vitamins was lower and depended on the agar content in FFs. Including vitamins C, B12 and D3 in the FFs structure did not protect them during storage. Therefore, the loss of vitamins during the technological process and the shelf-life of FFs must be considered when calculating product recipes.

**Table 5. Vitamin retention after the technological process and 6 weeks of storage.**

| Agar content in FF, % | Vitamins retention, % | |
|---|---|---|
| | During technological process | During storage for 6 weeks |
| **Vitamin C** | | |
| **0.8** | 50.6±1.2 A | 90.5±0.8 A |
| **1.0** | 47.5±0.9 B | 95.8±1.0 A |
| **1.5** | 51.4±1.3 A | 67.3±1.2 B |
| **Vitamin B9** | | |
| **0.8** | 126.7±43.2 A | 94.7±26.1 A |
| **1.0** | 115.0±31.5 A | 94.5±21.2 A |
| **1.5** | 116.7±28.8 A | 95.7±19.4 A |
| **Vitamin B12** | | |
| **0.8** | 140.0±30.0 A | 78.6±15.2 A |
| **1.0** | 130.0±28.1 A | 88.5±15.4 A |
| **1.5** | 127.5±21.6 A | 86.3±19.6 A |
| **Vitamin D3** | | |
| **0.8** | 135.0±7.1 A | 90.0±8.8 A |
| **1.0** | 96.0±9.5 B | 93.5±5.4 B |
| **1.5** | 129.0±9.9 A | 78.7±7.9 A |

*Values are presented as mean±standard deviation; Uppercase letters indicate significant (p<0.05) differences in the characteristics of FF with different agar content*

Vitamins release kinetics during *in vitro* digestion. The release kinetics of vitamins added into the formulation of FFs were also measured. These data are presented in Table 6 as the ratio of the amount of individual vitamins found in the liquid digestive phase to the amount of compound incorporated into the formulation of FFs. To see the effect of gel structure on the release kinetics of vitamins as a control non-gelled formulation was used.

**Table 6. Vitamins release kinetics from FFs during in-vitro digestion.**

| Sample | Vitamin release in the gastric (G) and intestinal (D) phases, % | | | | | |
|---|---|---|---|---|---|---|
| | G0 | G60 | G120 | G180 | D60 | D120 |
| **Vitamin C** | | | | | | |
| **Control** | 81.8 ±8.3 Aa | 78.9 ±5.9 Aa | 79.7 ±5.4 Aa | 77.9 ±6.4 Aa | 84.3 ±4.3 Aa | 80.2 ±4.4 Aa |
| **1.5% agar** | 46.8 ±9.6 Ba | 52.1 ±8.4 Bb | 47.5 ±6.2 Bb | 44.1 ±7.3 Bb | 68.7 ±4.1 Bc | 70.7 ±5.2 Ac |
| **Vitamin B9** | | | | | | |
| **Control** | 79.4 ±2.8 Aa | 75.5 ±2.6 Aa | 62.6 ±8.6 Aa | 75.9 ±6.9 Aa | 88.1 ±7.7 Aa | 85.6 ±6.8 Aa |
| **1.5% agar** | 31.2 ±8.1 Ba | 36.9 ±6.1 Ba | 39.3 ±5.1 Ba | 40.9 ±9.9 Aa | 67.3 ±7.2 Bb | 77.5 ±4.8 Ab |
| **Vitamin B12** | | | | | | |
| **Control** | 88.9 ±7.2 Aa | 85.6 ±8.3 Aa | 87.5 ±3.2 Aa | 89.7 ±8.3 Aa | 95.3 ±5.1 Aa | 99.6 ±6.0 Aa |

(continued)

| Vitamin B12 | | | | | | |
|---|---|---|---|---|---|---|
| 1.5% agar | 47.8 ±6.9 Ba | 75.1 ±4.0 Ab | 88.9 ±5.2 Ac | 81.0 ±4.9 Ac | 77.5 ±8.3 Bc | 79.8 ±13.2 Ac |
| **Vitamin D3** | | | | | | |
| | | | | G180 | | D120 |
| Control | - | - | - | 85.6 ±4.9 Aa | - | 105.5 ±7.3 Ab |
| 1.5% agar | - | - | - | 45.7 ±5.5 Ba | - | 110.2 ±7.6 Ab |
| *Different letters denote statistically significant differences ($p \leq 0.05$) between means: uppercase letters within control and 1.5% agar samples, lowercase letters within points of digestion.* | | | | | | |

During the digestion period of gelled FFs and non-gelled control, significant differences were found between the release of all vitamins added. Vitamin release began immediately after adding gastric fluids (G0) to the samples and remained at the same level throughout the gastric period. At the beginning of the intestinal phase (D60), vitamin release was significantly increased, but further incubation with intestinal fluids no longer affected the release of vitamins. At the end of the duodenal phase, 70 - 100% of the vitamins were released.

However, the release kinetics of vitamins during in vitro digestion from gelled and non-gelled samples differ. The significantly lower rate of vitamins excreted from non-gelled samples in comparison with gelled samples was determined under gastric conditions. At G180, 77.9±6.4 % of the added vitamin C was released from the control sample, and 44.1±7.3 % - from the gelled sample. At the end of the intestinal phase (D120), the amount of released vitamin C was 80.2±4.4 from the non-gelled samples and 70.7±5.2 from the gelled sample. The release of vitamins B9 and B12 showed very similar trends.

During the gastric phase, 85.6±4.9 and 45.7±5.5 % of vitamin D3 were released from the non-gelled and gelled samples, respectively. The release of this vitamin continued to increase during the intestinal phase and was about 100 % at the end of digestion for both samples. Such results conclude that the gel structure of FFs can limit the excretion of vitamin C, B9, B12, and vitamin D3 in the gastrointestinal stage and ensure the excretion of vitamins in the range of 70-100 % in the intestinal digestion stage.

LIST OF NON-PATENT CITATIONS

[1] M. Frangeskou, B. Lopez-Valcarcel, Lluis Serra-Majem. Dehydration in the elderly: A review focused on economic burden. The Journal of nutrition, health and aging, 2015, Vol 19, Issue 6, 619-627

[2] Michael A. Via MD, Jeffrey I. Mechanick MD. Malnutrition, Dehydration, and Ancillary Feeding Options in Dysphagia Patients. Otolaryngologic Clinics of North America, 2013, Vol 46, Issue 6, 1059-1071

[3] Waters, A. M., Patterson, J., Bhat, P. r, & Phillips, A. W. (2022). Investigating dysphagia in adults: Symptoms and tests. BMJ, 379, Article e067347. https://doi.org/10.1136/ bmj2021067347

[4] Thiyagalingam, S., Kulinski, A. E., Thorsteindottir, B., Schindelar, K. L., & Takahashi, P. Y. (2021). Dysphagia in older adults. Mayo Clin. Proc., 96(2), 488-497

[5] P. Viñas, M. Bolivar-Prados, N. Tomsen, A. Costa, S. Marin, N. Barcons, P. Clavé. Prevalence of dehydration among adult patients with Oropharyngeal Dysphagia: A systematic and scoping review. Clinical Nutrition ESPEN, 2023, 54, 566-580.

[6] O'Keeffe, S.T. Use of modified diets to prevent aspiration in oropharyngeal dysphagia: Is current practice justified? BMC Geriatr. 2018, 18, 167.

[7] Newman, R.; Vilardell, N.; Clavé, P.; Speyer, R. Effect of Bolus Viscosity on the Safety and Efficacy of Swallowing and the Kinematics of the Swallow Response in Patients with Oropharyngeal Dysphagia: White Paper by the European Society for Swallowing Disorders (ESSD). Dysphagia 2016, 31, 232-249

[8] Vivanti, A.P.; Campbell, K.L.; Suter, M.S.; Hannan-Jones, M.T.; Hulcombe, J. A. A. Contribution of thickened drinks, food and enteral and parenteral fluids to fluid intake in hospitalised patients with dysphagia. J. Hum. Nutr. Diet 2009, 22, 148-155

[9] Berkhout, M. M., Cools, H. J. M., & Van Houwelingen, H. C. The relationship between difficulties in feeding oneself and loss of weight in nursing-home patients with dementia. Age and Ageing, 1998, 27(5), 637-641

[10] Pouyet V. et al. Attractiveness and consumption of finger foods in elderly Alzheimer's disease patients. Food Quality and Preference 34, 2014, 62-69

[11] Sarah Forsberg, Maria Nyberg, Viktoria Olsson, Elisabet Rothenberg, Wender L.P. Bredie, Karin Wendin & Albert Westergren (2024) Finger Food Meals as a Means of Improving Mealtimes for People with Motoric Eating Difficulties: A Pilot Study, Journal of Nutrition in Gerontology and Geriatrics, 43:2, 95-115, DOI: 10.1080/21551197.2024.2358755

[12] Volkert D, Beck AM, Cederholm T, et al. ESPEN guideline on clinical nutrition and hydration in geriatrics. Clin Nutr. 2019;38(1):10-47. doi:10.1016/j.clnu.2018.05.024

[13] J.L. Murphy, J. Holmes, C. Brooks. Nutrition and dementia care: developing an evidence-based model for nutritional care in nursing homes BMC Geriatr., 17 (55) (2017), 10.1186/s12877-017-0443-2

[14] Forsberg S, Olsson V, Verstraelen E, Krona A, Bredie WLP & Wendin K. (2022). Proposal of development of finger foods for older adults with motoric eating difficulties - A study based on creative design. International Journal of Gastronomy and Food Science, 28, 100516. https://doi.org/10.1016/j.ijgfs.2022.100516

[15] Kurapkiene, Aušrinė; Vinauskiene, Rimante; Jasutiene, Ina; Damulevičienė, Gyte; Knašienė, Jurgita; Lesauskaite, Vita; Sulmont-Rosse, Claire; Eisinaite, Viktorija; Leskauskaite, Daiva. One-bite sized 3D printed finger foods, oriented to malnutrition, sarcopenia, and frailty prevention in the older people // Journal of the science of food and agriculture. 2024, vol. 104, iss. 10, p. 6289-6297. DOI: 10.1002/jsfa.13463.

[16] Kurapkiene, Aušrinė; Vinauskiene, Rimante; Jasutiene, Ina; Keršienė, Milda; Damulevičienė, Gyte; Knašienė, Jurgita; Lesauskaite, Vita; Sulmont-Rossé, Claire; Eisinaite, Viktorija; Leskauskaite, Daiva. Bigel as a curcumin delivery system and its application in 3D-printed in-between-meal foods to boost the immune system of elderly people // Food bioscience. Amsterdam : Elsevier. ISSN 2212-4292. eISSN 2212-4306. 2024, vol. 61, art. no. 104789, p. 1-8. DOI: 10.1016/j.fbio.2024.104789.

[17] J.A. Cichero, P. Lam, C.M. Steele, B. Hanson, J. Chen, R.O. Dantas, et al. Development of international terminology and definitions for texture-modified foods and thickened fluids used in dysphagia management: The IDDSI framework, Dysphagia, 32 (2) (2017), pp. 293-31

[18] K. Nishinari, M. Turcanu, M. Nakauma, Y. Fang. Role of fluid cohesiveness in safe swallowing NPJ Science of Food, 3 (2019), p. 5.

**Claims**

1. A method of producing a food product, comprising at least steps of:

   a) selecting and preparing essential components, or food ingredients comprising said components, said components at least

   - water of 95-97 weight %;
   - agar of 0.8-1.5 weight %;
   - Sodium carboxymethylcellulose of 0.5-0.6 weight %;
   - mono- and di-saccharides 2-3 weight %;
   - organic acids - 0.3-0.5 weight %;

   b) preparing a liquid formulation by blending the selected components or ingredients;
   c) adjusting the pH of the blended formulation to pH 3.1-3.2;
   d) treating the formulation by heat, for dissolving the components and ensuring microbial safety;
   e) shape the liquid formulation and allow gel formation from it.

2. The method according to claim 1, comprising a further step of
   f) packaging and storage of the obtained finger-food product.

3. The method according to any of claims 1-2, further comprising steps of:

   d1) after the step d) - cooling down the heated formulated to the temperature suitable to blend vitamins into the formulation;
   d2) after the step d1) - blending one or more vitamins into the formulation, any one or a composition of: C, B9, B12 and D3.

4. The method according to any of claims 1-3, further comprising

   • selecting supplementary nutritional components, any one of zinc, iron, selenium, and
   • blending supplementary nutritional components into the liquid formulation, together with other dry components, preferably, before the step **d)** of the formulation treatment by heat.

5. The method according to any of claims 1-4, further comprising

• selecting food preservative components,

  ◦ Potassium sorbate of 0.05 - 0.1 weight %;
  ◦ Sodium benzoate of 0.01 to 0.05 % weight %;

• blending the selected preservative components into the liquid formulation, together with other dry components, preferably, before the step **d)** of the formulation treatment by heat.

6. The method according to any of claims 1-5, further comprising

• selecting food flavors and/or colour agents, preferably, of natural origin;
• blending the selected food flavors and colour agents into the liquid formulation, together with other dry components, preferably, before the step **d)** of the formulation treatment by heat.

7. The method according to any of claims 1-6, wherein the organic acids of 0.3-0.5 wt% are any one of citric, malic, quinic acids, or any combination thereof.

8. The method according to any of claims 1-7, wherein the pH adjustment of the formulation in step c) is done by adding citric acid into the blended formulation.

9. The method according to any of claims 1-8, wherein in the step **e)** the formulation is shaped into a one-bite finger food shaped-mold, and kept at temperature of 2-6 °C until a gel forms and not less than 12 hours.

10. The method according to any of claims 1-9, wherein in the step **a)** the selected essential components of the formulation obtainable are:

- water of 71-78 weight %;
- agar of 0.8-1.5 weight %;
- sodium carboxymethylcellulose of 0.5-0.6 weight %, and
- juice concentrate conditioned to substitute a part of the formulation's required water to the total 95-97 weight %, mono- and di-saccharides 2 - 3 weight%, and organic acids - 0.3-0.5 weight %.

11. The method according to any of claims 1-10, wherein in the step **c)** the formulation treatment by heat is done at 98 - 100 °C up to and not more 5 minutes.

12. A food product obtainable by the method according to any one of claims 1-11.

13. The food product according to claim 12, **characterized in that** the food product has the following measurable physical charateristics:

• pH is within the range 3.1 - 3.2;
• shear viscosity at 50 s$^{-1}$ at least 1000 cP;
• hardness in the range 110 - 450 g.

14. The food product according to to any of claims 12-13, **characterized in that** the product complies to the IDDSI category levels of the International Dysphagia Diet Standardisation Initiative 2019, having shape-retaining gel texture with the description of level 6 soft and bite-sized foods and has a viscosity of greater than 1000 cP.

15. Use of the food product according to any one of claims 12-14, by elderly people and dysphagia patients, for at least reducing their dehydration and aspiration pneumonia risks.

**Amended claims in accordance with Rule 137(2) EPC.**

1. A method of producing a food product, comprising at least steps of:

a) selecting and preparing essential components, or food ingredients comprising said components, said components at least

- water of 95-97 weight %;
- agar of 0.8-1.5 weight %;
- sodium carboxymethylcellulose of 0.5-0.6 weight %;
- mono- and di-saccharides 1.2 to 2 weight %;
- organic acids - 0.5 to 0.9 weight %;
- optionally, any of vitamins, preservative components, food flavors and colour agents, in their whole amount from 0 to 2 weight %, to complete 100w% of a formulation to be prepared;

b) preparing the liquid formulation by blending the selected components or ingredients;

c) adjusting the pH of the blended formulation to pH 3.1-3.2, wherein if needed, the pH adjusted by adding citric acid until the pH of the formulation reaches pH 3.1 - 3.2;

d) treating the formulation by heat, for dissolving the components and ensuring microbial safety;

e) shape the liquid formulation to a finger-food shape and allow gel formation from it thereby obtaining the finger-food product.

2. The method according to claim 1, comprising a further step of
f) packaging and storage of the obtained finger-food product.

3. The method according to any of claims 1-2, further comprising steps of:

d1) after the step d) - cooling down the heated formulated to the temperature suitable to blend vitamins into the formulation;

d2) after the step d1) - blending one or more vitamins into the formulation, any one or a composition of: C, B9, B12 and D3.

4. The method according to any of claims 1-3, further comprising

• selecting supplementary nutritional components, any one of zinc, iron, selenium, and
• blending supplementary nutritional components into the liquid formulation, together with other dry components, preferably, before the step d) of the formulation treatment by heat.

5. The method according to any of claims 1-4, further comprising

• selecting food preservative components,

○ Potassium sorbate of 0.05 - 0.1 weight %;
○ Sodium benzoate of 0.01 to 0.05 weight %;

• blending the selected preservative components into the liquid formulation, together with other dry components, preferably, before the step d) of the formulation treatment by heat.

6. The method according to any of claims 1-5, further comprising

• selecting food flavors and/or colour agents, preferably, of natural origin;
• blending the selected food flavors and colour agents into the liquid formulation, together with other dry components, preferably, before the step d) of the formulation treatment by heat.

7. The method according to any of claims 1-6, wherein the organic acids of 0.5 to 0.9 wt% are any one of citric, malic, quinic acids, or any combination thereof.

8. The method according to any of claims 1-7, wherein the pH adjustment of the formulation to pH 3.1 - 3.2 in step c) is done by adding citric acid into the blended formulation.

9. The method according to any of claims 1-8, wherein in the step e) the formulation is shaped into a one-bite finger food shaped-mold, and kept at temperature of 2-6 °C until a gel forms and not less than 12 hours.

10. The method according to any of claims 1-9, wherein in the step a) the selected essential components of the formulation obtainable are:

> - water of 71-78 weight %;
> - agar of 0.8-1.5 weight %;
> - sodium carboxymethylcellulose of 0.5-0.6 weight %, and
> - juice concentrate conditioned to substitute a part of the formulation's required water to the total 95-97 weight %, mono- and di-saccharides 1.2 to 2 weight %, and organic acids - 0.5 to 0.9 weight %.

11. The method according to any of claims 1-10, wherein in the step c) the formulation treatment by heat is done at 98 - 100°C up to and not more 5 minutes.

12. A finger-food product obtainable by the method according to any one of claims 1-11.

13. The finger-food product according to claim 12, **characterized in that** the food product has the following measurable physical charateristics:

> • water contents in the range of 95-97 weight %;
> • pH is within the range 3.1 - 3.2;
> • shear viscosity at 50 $s^{-1}$ at least 1000 cP at 25°C;
> • food hardness of the finger-food product in the range 110 - 450 N.

14. The food product according to to any of claims 12-13, **characterized in that** the product complies to the IDDSI category levels of the International Dysphagia Diet Standardisation Initiative 2019, having shape-retaining gel texture with the description of level 6 soft and bite-sized foods and has a viscosity of greater than 1000 cP at 25°C.

15. Use of the food product according to any one of claims 12-14, by elderly people and dysphagia patients, for at least reducing their dehydration and aspiration pneumonia risks.

**Fig. 1**

| a) Finger food (FF) with 0.8 of agar | | |
|---|---|---|
| **(1) Initial view** | | |
| **(2) Fork pressure test** | | |
| **(3) Spoon tilt test** | | |
| **(4) Fork separation test** | | |

Fig. 2

| b) Finger food FF with 1.0 of agar | |
|---|---|
| (1) Initial view | |
| (2) Fork pressure test | |
| (3) Spoon tilt test | |
| (4) Fork separation test | |

Fig. 2 (continued)

| c) FF with 1.5 of agar | | | |
|---|---|---|---|
| **(1) Initial view** | | | |
| **(2) Fork pressure test** | | | |
| **(3) Spoon tilt test** | | | |
| **(4) Fork separation test** | | | |

Fig. 2 (continued)

**Fig. 3**

Fig. 3 (continued)

| | Low deformation | High deformation | Low deformation |
|---|---|---|---|
| FF with 0.8 % agar | | | |
| FF with 1.0 % agar | | | |
| FF with 1.5 % agar | | | |

Fig. 4

| Sample | Product storage duration, weeks | | |
| --- | --- | --- | --- |
| | 0 | 1 | 2 |
| | Product appearence | | |
| 0.8% agar | | | |
| 1.0% agar | | | |
| 1.5% agar | | | |
| Sample | Product storage duration, weeks | | |
| | 3 | 4 | 6 |
| 0.8% agar | | | |
| 1.0% agar | | | |
| 1.5% agar | | | |

Fig. 5

EUROPEAN SEARCH REPORT

**Application Number**

EP 24 22 2840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 168 980 A (QINGDAO LIBANGDA MARINE TECHNOLOGY CO LTD) 26 June 2013 (2013-06-26) | 12 | INV. A23L29/256 A23L29/262 |
| A | * claims; example 4 * | 1-11, 13-15 | A23L29/30 A23L33/125 |
| | ----- | | A23L33/16 |
| X,D | WO 2020/128531 A1 (JELLY DROPS LTD [GB]) 25 June 2020 (2020-06-25) * page 1, lines 15-22; claims 1,2,5,12,14,15,18; examples * * page 2, lines 16-28 * * page 17, line 27 - page 18 * | 1-15 | A23L33/00 A23L33/15 A23L21/15 |
| | ----- | | |
| A | WO 2024/134616 A1 (GELTEQ LTD [AU]; SCHILLER DOMINIC [GB]) 27 June 2024 (2024-06-27) * paragraph [042-]; claims; figure 1; examples * | 1-15 | |
| | ----- | | |
| A | WO 2004/010796 A1 (OTSUKA PHARMA CO LTD [JP]; TAKAICHI AKIHISA [JP] ET AL.) 5 February 2004 (2004-02-05) * page 24; claims 1,5,6 * | 1-15 | TECHNICAL FIELDS SEARCHED (IPC) A23L A61P |
| | ----- | | |
| A | EP 1 046 347 A1 (OTSUKA FOOD CO LTD [JP]) 25 October 2000 (2000-10-25) * claims 1-12; examples * | 1-15 | |
| | ----- | | |

The present search report has been drawn up for all claims

| Place of search | Date of completion of the search | Examiner |
|---|---|---|
| Munich | 12 June 2025 | Muller, Isabelle |

EPO FORM 1503 03.82 (P04C01)

2

## ANNEX TO THE EUROPEAN SEARCH REPORT
## ON EUROPEAN PATENT APPLICATION NO.

EP 24 22 2840

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2025

| Patent document cited in search report | | Publication date | Patent family member(s) | | Publication date |
|---|---|---|---|---|---|
| CN 103168980 | A | 26-06-2013 | NONE | | |
| WO 2020128531 | A1 | 25-06-2020 | NONE | | |
| WO 2024134616 | A1 | 27-06-2024 | GB | 2625768 A | 03-07-2024 |
| | | | WO | 2024134616 A1 | 27-06-2024 |
| WO 2004010796 | A1 | 05-02-2004 | AT | E326149 T1 | 15-06-2006 |
| | | | CA | 2489566 A1 | 05-02-2004 |
| | | | CN | 1671300 A | 21-09-2005 |
| | | | DE | 60305331 T2 | 01-02-2007 |
| | | | EP | 1541042 A1 | 15-06-2005 |
| | | | ES | 2261983 T3 | 16-11-2006 |
| | | | JP | WO2004010796 A1 | 02-12-2005 |
| | | | KR | 20050033635 A | 12-04-2005 |
| | | | TW | I324047 B | 01-05-2010 |
| | | | US | 2005260322 A1 | 24-11-2005 |
| | | | WO | 2004010796 A1 | 05-02-2004 |
| EP 1046347 | A1 | 25-10-2000 | AU | 754277 B2 | 07-11-2002 |
| | | | CA | 2318566 A1 | 15-07-1999 |
| | | | CN | 1284838 A | 21-02-2001 |
| | | | DE | 69820119 T2 | 08-07-2004 |
| | | | EP | 1046347 A1 | 25-10-2000 |
| | | | ES | 2210850 T3 | 01-07-2004 |
| | | | ID | 25929 A | 09-11-2000 |
| | | | JP | 3648597 B2 | 18-05-2005 |
| | | | KR | 20010040325 A | 15-05-2001 |
| | | | TW | 542700 B | 21-07-2003 |
| | | | US | 6458395 B1 | 01-10-2002 |
| | | | WO | 9934690 A1 | 15-07-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020128531 A1
- WO 2022208070 A1

**Non-patent literature cited in the description**

- **M. FRANGESKOU** ; **B. LOPEZ-VALCARCEL** ; **LLUIS SERRA-MAJEM**. Dehydration in the elderly: A review focused on economic burden. *The Journal of nutrition, health and aging*, 2015, vol. 19 (6), 619-627
- **MICHAEL A.** ; **VIA MD** ; **JEFFREY I.** ; **MECHANICK MD**. Malnutrition, Dehydration, and Ancillary Feeding Options in Dysphagia Patients. *Otolaryngologic Clinics of North America*, 2013, vol. 46 (6), 1059-1071
- **WATERS, A. M.** ; **PATTERSON, J.** ; **BHAT, P. R** ; **PHILLIPS, A. W.** Investigating dysphagia in adults: Symptoms and tests. *BMJ*, 2022, vol. 379, e067347, https://doi.org/10.1136/ bmj2021067347
- **THIYAGALINGAM, S.** ; **KULINSKI, A. E.** ; **THOR-STEINDOTTIR, B.** ; **SCHINDELAR, K. L.** ; **TAKA-HASHI, P. Y.** Dysphagia in older adults. *Mayo Clin. Proc.*, 2021, vol. 96 (2), 488-497
- **P. VIÑAS** ; **M. BOLIVAR-PRADOS** ; **N. TOMSEN** ; **A. COSTA** ; **S. MARIN** ; **N. BARCONS** ; **P. CLAVÉ**. Prevalence of dehydration among adult patients with Oropharyngeal Dysphagia: A systematic and scoping review. *Clinical Nutrition ESPEN*, 2023, vol. 54, 566-580
- **O'KEEFFE, S.T.** Use of modified diets to prevent aspiration in oropharyngeal dysphagia: Is current practice justified?. *BMC Geriatr.*, 2018, vol. 18, 167
- **NEWMAN, R.** ; **VILARDELL, N.** ; **CLAVÉ, P.** ; **SPEYER, R.** Effect of Bolus Viscosity on the Safety and Efficacy of Swallowing and the Kinematics of the Swallow Response in Patients with Oropharyngeal Dysphagia: White Paper by the European Society for Swallowing Disorders (ESSD). *Dysphagia*, 2016, vol. 31, 232-249
- **VIVANTI, A.P.** ; **CAMPBELL, K.L.** ; **SUTER, M.S.** ; **HANNAN-JONES, M.T.** ; **HULCOMBE, J. A. A**. Contribution of thickened drinks, food and enteral and parenteral fluids to fluid intake in hospitalised patients with dysphagia.. *J. Hum. Nutr. Diet*, 2009, vol. 22, 148-155

- **BERKHOUT, M. M.** ; **COOLS, H. J. M.** ; **VAN HOUWELINGEN, H. C.** The relationship between difficulties in feeding oneself and loss of weight in nursing-home patients with dementia. *Age and Ageing*, 1998, vol. 27 (5), 637-641
- **POUYET V. et al.** Attractiveness and consumption of finger foods in elderly Alzheimer's disease patients. *Food Quality and Preference*, 2014, vol. 34, 62-69
- **SARAH FORSBERG** ; **MARIA NYBERG** ; **VIKTOR-IA OLSSON** ; **ELISABET ROTHENBERG** ; **WEN-DER L.P. BREDIE** ; **KARIN WENDIN** ; **ALBERT WESTERGREN**. Finger Food Meals as a Means of Improving Mealtimes for People with Motoric Eating Difficulties: A Pilot Study. *Journal of Nutrition in Gerontology and Geriatrics*, 2024, vol. 43 (2), 95-115
- **VOLKERT D** ; **BECK AM** ; **CEDERHOLM T et al.** ESPEN guideline on clinical nutrition and hydration in geriatrics. *Clin Nutr.*, 2019, vol. 38 (1), 10-47
- **J.L. MURPHY** ; **J. HOLMES** ; **C. BROOKS**. Nutrition and dementia care: developing an evidence-based model for nutritional care in nursing homes. *BMC Geriatr.*, 2017, vol. 17 (55)
- **FORSBERG S** ; **OLSSON V** ; **VERSTRAELEN E** ; **KRONA A** ; **BREDIE WLP** ; **WENDIN K**. Proposal of development of finger foods for older adults with motoric eating difficulties - A study based on creative design. *International Journal of Gastronomy and Food Science*, 2022, vol. 28, 100516, https://doi.org/10.1016/j.ijgfs.2022.100516
- **J.A. CICHERO** ; **P. LAM** ; **C.M. STEELE** ; **B. HANSON** ; **J. CHEN** ; **R.O. DANTAS et al.** Development of international terminology and definitions for texture-modified foods and thickened fluids used in dysphagia management: The IDDSI framework. *Dysphagia*, 2017, vol. 32 (2), 293-31
- **K. NISHINARI** ; **M. TURCANU** ; **M. NAKAUMA** ; **Y. FANG**. Role of fluid cohesiveness in safe swallowing. *NPJ Science of Food*, 2019, vol. 3, 5